(19) Europäisches Patentamt
European Patent Office
Office européen des brevets

(11) EP 3 162 809 B1

(12) **EUROPEAN PATENT SPECIFICATION**

(45) Date of publication and mention of the grant of the patent:
**04.08.2021 Bulletin 2021/31**

(21) Application number: **15812576.5**

(22) Date of filing: **25.06.2015**

(51) Int Cl.:
***C07K 1/22*** *(2006.01)* ***B01D 15/08*** *(2006.01)*
***B01J 20/24*** *(2006.01)* ***G01N 30/26*** *(2006.01)*
***G01N 30/88*** *(2006.01)* ***C07K 16/00*** *(2006.01)*
***B01J 20/26*** *(2006.01)* ***B01J 20/32*** *(2006.01)*
***B01J 20/28*** *(2006.01)*

(86) International application number:
**PCT/JP2015/068403**

(87) International publication number:
**WO 2015/199196 (30.12.2015 Gazette 2015/52)**

(54) **CARRIER FOR AFFINITY CHROMATOGRAPHY**

TRÄGER FÜR AFFINITÄTSCHROMATOGRAFIE

SUPPORT POUR CHROMATOGRAPHIE D'AFFINITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2014 JP 2014132611**

(43) Date of publication of application:
**03.05.2017 Bulletin 2017/18**

(73) Proprietors:
- **JSR Corporation**
  **Minato-ku**
  **Tokyo 105-8640 (JP)**
- **JSR Life Sciences Corporation**
  **Tokyo 105-8640 (JP)**

(72) Inventors:
- **MOMIYAMA, Masaki**
  **Tokyo 105-8640 (JP)**
- **TSUDA, Sachiko**
  **Tokyo 105-8640 (JP)**
- **NORINOBU, Tomoya**
  **Tokyo 105-8640 (JP)**

(74) Representative: **TBK**
**Bavariaring 4-6**
**80336 München (DE)**

(56) References cited:
**EP-A1- 2 339 339**
**WO-A1-2007/142331**
**WO-A1-2011/125673**
**WO-A1-2011/125674**
**US-A1- 2014 005 357**

- **None**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to an affinity chromatography carrier. Specifically, the present invention relates to an affinity chromatography carrier useful for purification of proteins such as antibodies.

### Background Art

Affinity chromatography has an important role in the research, development, and production of proteins including monoclonal antibodies. An affinity chromatography carrier generally includes a solid support having a ligand capable of selectively binding to a target material. Affinity chromatography, which uses a solid support on which a ligand has high selectivity to a target material, enables high-speed, economical purification with high yield as compared with other chromatographic techniques such as ion chromatography, gel filtration chromatography, and reversed-phase liquid chromatography.

In general, affinity chromatography for protein purification is required to have, for example, the following performance characteristics: (1) not to cause nonspecific adsorption of impurities other than target proteins; (2) not to cause a decrease in binding capacity due to long-term storage and to have high storage stability; and (3) to have mechanical strength enough for column operation.

Under these circumstances, agarose particles (Patent Literatures 1 and 2), porous particles (Patent Literatures 3 and 4) including a styrene-divinylbenzene copolymer, and porous particles (Patent Literatures 5 and 6) including a polymer of a methacrylate-based vinyl monomer are proposed as solid supports for affinity chromatography carrier.

### Citation List

### Patent Literatures

Patent Literature 1: JP 2008-523140 W
Patent Literature 2: JP 2009-522580 W
Patent Literature 3: JP 08-278299 A
Patent Literature 4: JP 10-501173 W
Patent Literature 5: WO 2011/125674 A
Patent Literature 6: JP 2012-141212 A

US 2014/005357 A1 discloses a further support for affinity chromatography, containing an immobilized protein ligand. EP 2339339 A1 discloses an affinity chromatography packing material including porous mother particles that include a copolymer of a monomer mixture including a crosslinkable vinyl monomer and an epoxy group-containing vinyl monomer, a ligand being bound to the porous mother particles, and the porous mother particles including a ring-opening epoxy group produced by ring-opening of the epoxy group included in the porous mother particles.

### Summary of Invention

### Technical Problem

Meanwhile, agarose particles, which have generally low elastic modulus, have the disadvantage that it can increase the pressure in a column when a medium flows at a high rate through the column. In addition, agarose particles are not easily available with constant quality because they are generally produced from natural seaweed through a long complicated process.

Although generally having high alkali resistance, porous particles including a copolymer of aromatic vinyl monomers, such as a styrene-divinylbenzene copolymer, have low hydrophilicity and thus have the disadvantage that ligands on them have low activity so that the dynamic binding capacity for target materials is low.

Porous particles including a polymer of a methacrylate-based vinyl monomer have a high level of hydrophilicity and mechanical strength, whereas they are still required to have further improved ability to remove impurities.

An object to be achieved by the present invention is to provide an affinity chromatography carrier that suppresses nonspecific adsorption of impurities, suppresses a decrease in dynamic binding capacity due to long-term storage, and has high storage stability.

Solution to Problem

Thus, as a result of intensive studies, the inventors have accomplished the present invention based on the finding that a specific affinity chromatography carrier including: a solid support containing a copolymer including specific amounts of structural units derived from an epoxy group-containing monovinyl monomer and a polyvinyl monomer, respectively; a ligand coupled to the solid support; and ring-opened epoxy groups can have high dynamic binding capacity for target materials, a high ability to remove impurities, and high storage stability.
Specifically, the present invention provides <1> an affinity chromatography carrier as defined in claim 1.
The present invention also provides <2> an affinity chromatography column including: a column vessel; and the affinity chromatography carrier according to item <1> with which the column vessel is packed.
The present invention further provides <3> a method for purifying a target material, the method including using the affinity chromatography carrier according to item <1>. Advantageous Effects of Invention
The affinity chromatography carrier of the present invention reduces a residual amount of epoxy groups on the surface, and also has high hydrophilicity due to the ring-opened epoxy groups.
Therefore, according to the affinity chromatography carrier of the present invention, it is possible to improve an ability to remove nonspecifically adsorbing impurities, such as host cell proteins (HCPs) and DNA.
The affinity chromatography carrier of the present invention also allows the ligand to maintain high activity. Therefore, even when used in purification of, for example, immunoglobulin and then repeatedly used for a long period of time, the affinity chromatography carrier of the present invention suppresses a decrease in dynamic binding capacity for immunoglobulin and the like, which makes it possible to purify immunoglobulin at low cost.

Description of Embodiments

Hereinafter, the affinity chromatography carrier of the present invention will be described in detail.

[Affinity chromatography carrier]

<Composition of solid support>

The solid support constituting the affinity chromatography carrier of the present invention includes a copolymer including (M-1) 40 parts by mass or more to 80 parts by mass or less of a structural unit derived from an epoxy group-containing monovinyl monomer with respect to 100 parts by mass of all structural units and (M-2) 15 to 60 parts by mass of a structural unit derived from a polyvinyl monomer with respect to 100 parts by mass of all structural units, and also has ring-opened epoxy groups obtained by subjecting the epoxy groups to the ring-opening, wherein the copolymer further comprises (M-3) 5 parts by mass or more and 25 parts by mass or less of a structural unit derived from an epoxy-free monovinyl monomer with respect to 100 parts by mass of all structural units, wherein the epoxy-free monovinyl monomer is selected from the group consisting of ethylvinylbenzene, glycerol mono(meth)acrylate and hydroxyethyl(meth)acrylamide. The solid support may be in the form of, for example, any of a monolith, a membrane, a hollow fiber, particles, a cassette, and chips. Preferably, the solid support is in the form of particles. In order to increase surface area, the solid support is preferably in the form of porous materials such as porous particles. The porous particles are also preferably porous polymer particles. Besides the copolymer, the solid support may also contain a natural polymer, a synthetic polymer or the like.

((M-1) Structural unit derived from epoxy group-containing monovinyl monomer)

The epoxy group-containing monovinyl monomer is a monomer having, per molecular, one polymerizable vinyl group (ethylenically unsaturated bond-containing group) and one or more epoxy groups. The epoxy group-containing vinyl monomer can be used to introduce a suitable amount of epoxy groups into the solid support so that suitable amounts of coupled ligands and ring-opened epoxy groups can be obtained.
Examples of the epoxy group-containing monovinyl monomer include hydroxyl-free (meth)acrylates such as glycidyl (meth) acrylate, 4-hydroxybutyl (meth) acrylate glycidyl ether, 3,4-epoxycyclohexylmethyl (meth)acrylate, and $\alpha$-(meth)acryl-$\omega$-glycidyl polyethylene glycol; hydroxy group-containing (meth)acrylates such as glycerin mono(meth)acrylate glycidyl ether; aromatic monovinyl compounds such as vinylbenzyl glycidyl ether; and others such as allyl glycidyl ether, 3,4-epoxy-1-butene, and 3,4-epoxy-3-methyl-1-butene. These may be used alone or in combination of two or more.
Among these epoxy group-containing monovinyl monomers, in view of anti-fouling properties, storage stability or the like, epoxy group-containing (meth)acrylates and epoxy group-containing aromatic monovinyl compounds are preferred, and epoxy group-containing (meth) acrylates are more preferred. Glycidyl (meth)acrylate and 4-hydroxybutyl (meth)acrylate glycidyl ether are further preferred, and glycidyl (meth)acrylate is particularly preferred. When the solid

support is produced in the form of particles by normal phase suspension polymerization, the epoxy group-containing monovinyl monomer is preferably water-insoluble so that the amount of ring-opened epoxy groups can be easily controlled. In this regard, the term "water-insoluble" means that 10 g or more of the monomer is not completely soluble in 100 mL of water at room temperature (25°C). The epoxy group-containing monovinyl monomer used may be a commercially available product or synthesized in accordance with known methods.

The content of the structural unit derived from the epoxy group-containing monovinyl monomer is from 40 parts or more by mass to 80 parts by mass or less with respect to 100 parts by mass of all structural units. If the content is 20 parts by mass or less, the amount of ring-opened epoxy groups will be relatively small so that the affinity chromatography carrier after ligand coupling can have low hydrophilicity and low ability to remove impurities . On the other hand, if the content is more than 99.5 parts by mass, the affinity chromatography carrier can have low mechanical strength and fail to withstand handling.

In view of anti-fouling properties, storage stability, and other properties, the content of the structural unit derived from the epoxy group-containing monovinyl monomer is 40 parts by mass or more, further more preferably 50 parts by mass or more, with respect to 100 parts by mass of all structural units. In view of anti-fouling properties, storage stability or the like, the content of the structural unit derived from the epoxy group-containing monovinyl monomer is 80 parts by mass or less, with respect to 100 parts by mass of all structural units.

((M-2) Structural unit derived from polyvinyl monomer)

The polyvinyl monomer is a vinyl monomer having two or more polymerizable vinyl groups (ethylenically unsaturated bond-containing groups) per one molecule. Hereinafter, the polyvinyl monomer will be described, being categorized into a hydroxyl-free polyvinyl monomer and a hydroxy group-containing polyvinyl monomer. It will be understood that a single polyvinyl monomer may be used or two or more polyvinyl monomers may be used in combination.

Examples of the hydroxyl-free polyvinyl monomer include (meth)acrylates such as ethylene glycol di(meth)acrylate, diethylene glycol di(meth)acrylate, triethylene glycol di(meth)acrylate, tetraethylene glycol di(meth)acrylate, propylene glycol di(meth)acrylate, dipropylene glycol di(meth)acrylate, tripropylene glycol di(meth)acrylate, tetrapropylene glycol di(meth)acrylate, 1,4-butanediol di(meth)acrylate, 1,6-hexanediol di(meth)acrylate, trimethylolpropane tri(meth)acrylate, and pentaerythritol tetra(meth)acrylate; aromatic polyvinyl compounds such as divinylbenzene and trivinylbenzene; and allyl compounds such as butadiene, diallyl isocyanurate, and triallyl isocyanurate, which may be used alone or in combination of two or more. Among these hydroxyl-free polyvinyl monomers, (meth)acrylates and aromatic polyvinyl compounds are preferred.

The hydroxyl-free polyvinyl monomer preferably has 2 to 5 polymerizable vinyl groups per one molecule, more preferably 2 or 3 polymerizable vinyl groups per molecular.

Preferred examples of the hydroxy group-containing polyvinyl monomer include (meth) acrylic esters of polyalcohols, di- or poly-substituted (meth)acrylic esters of various saccharides, and polyalcohol (meth)acrylamides.

Examples of (meth) acrylic esters of polyalcohols include glycerin di(meth)acrylate, trimethylolethane di(meth)acrylate, trimethylolpropane di(meth)acrylate, butanetriol di(meth)acrylate, pentaerythritol di(meth)acrylate, pentaerythritol tri(meth)acrylate, dipentaerythritol di(meth)acrylate, dipentaerythritol tri(meth)acrylate, dipentaerythritol tetra(meth)acrylate, dipentaerythritol penta(meth)acrylate, inositol di(meth)acrylate, inositol tri(meth)acrylate, and inositol tetra(meth)acrylate.

Examples of di- or poly-substituted (meth) acrylic esters of various saccharides include glucose di(meth)acrylate, glucose tri(meth)acrylate, glucose tetra(meth)acrylate, mannitol di(meth)acrylate, mannitol tri(meth)acrylate, mannitol tetra(meth)acrylate, and mannitol penta(meth)acrylate.

Besides those listed above, examples of the hydroxy group-containing polyvinyl monomer also include dehydration condensation reaction products of (meth)acrylic acid and an aminoalcohol such as diaminopropanol, trishydroxymethylaminomethane, or glucosamine.

The hydroxy group-containing polyvinyl monomer preferably has 2 to 5 polymerizable vinyl groups per one molecule, more preferably 2 or 3 polymerizable vinyl groups per one molecule.

Among these polyvinyl monomers, hydroxyl-free polyvinyl monomers are preferred because they can be used in a relatively small amount to achieve high porosity and mechanical strength and are less likely to restrict the amount of the epoxy group-containing monovinyl monomer used. Among the hydroxyl-free polyvinyl monomers, (meth)acrylic esters with three polymerizable vinyl groups and aromatic polyvinyl compounds with two or three polymerizable vinyl groups are particularly preferred in view of anti-fouling properties, storage stability, and other properties. Particularly preferred examples include trimethylolpropane tri(meth)acrylate, divinylbenzene, and trivinylbenzene.

The content of the structural unit derived from the polyvinyl monomer is from 15 to 60 parts by mass with respect to 100 parts by mass of all structural units. If the content of the structural unit derived from the polyvinyl monomer is less than 0.5 parts by mass, the solid support will have low mechanical strength so that the affinity chromatography carrier can fail to withstand handling. If it is more than 80 parts by mass, the carrier will have reduced hydrophilicity, which can

make it impossible to obtain the effect of reducing HCPs, and the ligand will have reduced activity.

In view of anti-fouling properties, storage stability, or the like, the content of the structural unit derived from the polyvinyl monomer is 15 parts by mass or more, further more preferably 20 parts by mass or more, still more preferably 25 parts by mass or more, with respect to 100 parts by mass of all structural units. In view of anti-fouling properties, storage stability, or the like, the content of the structural unit derived from the polyvinyl monomer is 60 parts by mass or less, further more preferably 50 parts by mass or less, still more preferably 40 parts by mass or less, with respect to 100 parts by mass of all structural units.

((M-3) Structural unit derived from epoxy-free monovinyl monomer)

The copolymer in the solid support used in the present invention contains (M-3) 5 to 25 parts by mass of a structural unit derived from an epoxy-free monovinyl monomer with respect to 100 parts by mass of all structural units in addition to the structural units (M-1) and (M-2).

The epoxy-free monovinyl monomer is a vinyl monomer having one polymerizable vinyl group (ethylenically unsaturated bond-containing group) per one molecule and being free of any epoxy group. Hereinafter, the epoxy-free monovinyl monomer will be described, being categorized into an epoxy-free, hydroxyl-free, monovinyl monomer and an epoxy-free, hydroxy group-containing, monovinyl monomer.

The epoxy-free, hydroxyl-free, monovinyl monomer is a nonionic monomer in order to prevent nonspecific adsorption of impurities during purification. The nonionic monomer is ethylvinylbenzene.

The epoxy-free, hydroxy group-containing, monovinyl monomer is selected from the group consisting of glycerol mono(meth)acrylate and hydroxyethyl(meth)acrylamide, which may be used alone or in combination.

Among these epoxy-free monovinyl monomers, in view of anti-fouling properties or the like, epoxy-free, hydroxy group-containing, monovinyl monomers are preferred. That is, glycerol mono (meth) acrylate and hydroxyethyl(meth)acrylamide are preferred, and hydroxyethyl(meth)acrylamide is particularly preferred.

The content of the structural unit derived from the epoxy-free monovinyl monomer is 5 to 25 parts by mass or less with respect to 100 parts by mass of all structural units. When the content is 40 parts by mass or less, in the case of the hydroxy group-containing vinyl monomer, the resulting porous particles can have higher hydrophilicity, suppress aggregation, allow the ligand to have high activity, and have improved dynamic binding capacity for target materials. In addition, mechanical strength can also be improved. The content of the structural unit is more preferably 20 parts by mass or less, with respect to 100 parts by mass of all structural units. The content of the structural unit derived from the epoxy-free monovinyl monomer is 5 parts by mass or more with respect to 100 parts by mass of all structural units.

The combination of the contents of the structural units (M-1), (M-2), and (M-3) is a combination of 40 to 80 parts by mass of the structural unit (M-1), 15 to 60 parts by mass of the structural unit (M-2), and 5 to 25 parts by mass of the structural unit (M-3) with respect to 100 parts by mass of all structural units, even more preferably a combination of 40 to 80 parts by mass of the structural unit (M-1), 20 to 60 parts by mass of the structural unit (M-2), and 5 to 20 parts by mass of the structural unit (M-3) with respect to 100 parts by mass of all structural units.

(Epoxy group content)

The epoxy groups contained in the solid support before ligand coupling are functional groups for coupling to ligands and serving as a basis for improving the hydrophilicity of the affinity chromatography carrier after the ring opening. Before ligand coupling, the solid support preferably has an epoxy group content of 1 to 6 mmol/g, more preferably 2 to 5 mmol/g, even more preferably 2.5 to 4 mmol/g. When the solid support has an epoxy group content of 1 mmol/g or more before ligand coupling, a suitable amount of ring-opened epoxy groups can be obtained after ligand coupling, so that the ability to remove impurities other than target materials can be improved. When the solid support has an epoxy group content of 6 mmol/g or less before ligand coupling, the residual amount of epoxy groups after ligand coupling can be kept small, which can suppress the reduction in the ligand activity during the storage of the affinity chromatography carrier and can also suppress the reduction in the dynamic binding capacity for target materials during repeated use.

The epoxy group content of the solid support before ligand coupling can be controlled by the amount of the epoxy group-containing monovinyl monomer, the polymerization temperature, the polymerization time, the pH of the polymerization solution, the ring-opening process after the polymerization or the like.

<Production of solid support>

The solid support production method will be described with reference to an example where the solid support is in the form of porous particles.

The porous particles can be produced by, for example, known seed polymerization or suspension polymerization. The seed polymerization may be the two-stage swelling polymerization described in JP 57-24369 B. For the polymerization, if necessary, a polymerization initiator, a porosity-forming agent, an aqueous medium, a dispersion stabilizer, a surfactant, a polymerization modifier, a polymerization inhibitor, seed particles or the like may be used in addition to the monomers described above.

A preferred polymerization method for the production of the porous particles is a suspension polymerization method using an aqueous mixture containing a monomer mixture and a porosity-forming agent as essential components.

Specific methods for the suspension polymerization include, for example, a method that includes dissolving a polymerization initiator in a mixture solution (monomer solution) containing the monomer mixture and the porosity-forming agent, suspending the solution in an aqueous medium, and heating the suspension to a given temperature to polymerize the monomers; a method that includes dissolving a polymerization initiator in a mixture solution (monomer solution) containing the monomer mixture and the porosity-forming agent and adding the solution to an aqueous medium heated to a given temperature so that the monomers are polymerized; and a method that includes suspending, in an aqueous medium, a mixture solution (monomer solution) containing the monomer mixture and the porosity-forming agent, heating the suspension to a given temperature, and adding a polymerization initiator to the heated suspension so that the monomers are polymerized.

The polymerization initiator is preferably a radical polymerization initiator. The radical polymerization initiator may be, for example, an azo initiator, a peroxide initiator, or a redox initiator, specific examples of which include azobisisobutyronitrile, methyl azobisisobutyrate, azobis-2,4-dimethylvaleronitrile, benzoyl peroxide, di-tert-butyl peroxide, and benzoyl peroxide-dimethylaniline. The polymerization initiator is generally used in a total amount of about 0.01 to about 10 parts by mass with respect to 100 parts by mass of the total amount of the monomers.

The porosity-forming agent is used to produce porous particles. During polymerization in oil droplets, the porosity-forming agent exists together with the monomers and plays a role as a non-polymerizable component for forming pores. The porosity-forming agent may be of any type capable of being removed easily on the pore surface. The porosity-forming agent may be, for example, any of various organic solvents, a linear polymer soluble in the monomer mixture, or a combination thereof.

Examples of the porosity-forming agent include aliphatic hydrocarbons such as hexane, heptane, octane, nonane, decane, and undecane; alicyclic hydrocarbons such as cyclohexane and cyclopentane; aromatic hydrocarbons such as benzene, toluene, xylene, naphthalene, and ethylbenzene; halogenated hydrocarbons such as carbon tetrachloride, 1,2-dichloroethane, tetrachloroethane, and chlorobenzene; aliphatic alcohols such as butanol, pentanol, hexanol, heptanol, hexanol, 4-methyl-2-pentanol, and 2-ethyl-1-hexanol; alicyclic alcohols such as cyclohexanol; aromatic alcohols such as 2-phenylethyl alcohol and benzyl alcohol; ketones such as diethyl ketone, methyl isobutyl ketone, diisobutyl ketone, acetophenone, 2-octanone, and cyclohexanone; ethers such as dibutyl ether, diisobutyl ether, anisole, and ethoxybenzene; esters such as isopentyl acetate, butyl acetate, 3-methoxybutyl acetate, and diethyl malonate; as well as linear polymers such as homopolymers of a non-crosslinkable vinyl monomer. The porosity-forming agents may be used alone or in mixture of two or more.

The porosity-forming agent is generally used in a total amount of about 40 to about 400 parts by mass with respect to 100 parts by mass of the total amount of the monomers.

The aqueous medium may be, for example, an aqueous solution of a water-soluble polymer. The water-soluble polymer may be, for example, hydroxyethyl cellulose, polyvinyl alcohol, carboxymethyl cellulose, polyvinylpyrrolidone, starch, or gelatin. The aqueous medium is generally used in a total amount of about 200 to about 7,000 parts by mass with respect to 100 parts by mass of the total amount of the monomers.

When water is used as a dispersion medium for the aqueous medium, a dispersion stabilizer may also be used, such as sodium chloride, sodium sulfate, sodium carbonate, calcium carbonate, or calcium phosphate.

Any of various surfactants may also be used, including anionic surfactants such as alkyl sulfate salts, alkylaryl sulfate salts, alkyl phosphate salts, and fatty acid salts.

A polymerization inhibitor may also be used, such as a nitrite such as sodium nitrite, an iodide salt such as potassium iodide, tert-butylpyrocatechol, benzoquinone, picric acid, hydroquinone, copper chloride, or ferric chloride.

A polymerization modifier such as dodecyl mercaptan may also be used.

The polymerization temperature may be determined depending on the polymerization initiator. For example, when azobisisobutyronitrile is used as the polymerization initiator, the polymerization temperature is preferably from 50 to 100°C, more preferably from 60 to 90°C.

The polymerization time is generally from 5 minutes to 48 hours, preferably from 10 minutes to 24 hours.

After the polymerization is completed, washing with water and/or hot water is preferably performed to remove the adhering water-soluble polymer and other materials from the resulting porous particles.

Washing with a good solvent for the seed particles and/or the porosity-forming agent is preferred because it can make easy the ligand coupling described later. For example, acetone, ethanol, or isopropyl alcohol is preferably used as the washing solvent. If necessary, the porous particles may be dispersed using, for example, an ultrasonic disperser before or after the washing. In addition, decantation, filtration, sieve classification, or other methods for removing small particles and course particles are preferably performed in order to improve the pressure properties or the dynamic

binding capacity for target materials.

<Other features of affinity chromatography carrier than those described above>

(Ring-opened epoxy group)

The affinity chromatography carrier of the present invention has ring-opened epoxy groups, which are obtained by subjecting the epoxy groups to the ring-opening. The ring-opened epoxy groups can be obtained by subjecting the epoxy groups to the ring-opening that are contained in the copolymer after a ligand is coupled to the solid support including the copolymer. Namely, the ring-opened epoxy groups can be obtained by ring-opening of residual epoxy groups other than the epoxy groups coupled to the ligand. The epoxy groups may include not only those in the copolymer but also those introduced later from epichlorohydrin, a diglycidyl ether compound or the like. It does not matter at what stage the epoxy groups are introduced in the production of the affinity chromatography carrier of the present invention.
In the affinity chromatography carrier of the present invention, most of the epoxy groups on the surface of the solid support are in the form of ring-opening, so that the affinity chromatography carrier will release a significantly reduced amount of hydroxide ions when it is brought into contact with a high concentration of chloride ions so that the residual epoxy groups are ring-opened.
Therefore, the affinity chromatography carrier of the present invention is such that after a certain column vessel is packed with the affinity chromatography carrier and then purged with a 2 M NaCl-containing 20 mM sodium phosphate buffer with a pH of 7.5, allowing the column to stand at 40°C for 7 days, an increase of the pH in the column is 2 or less. This feature allows the affinity chromatography carrier to have a high ability to remove impurities and to have high storage stability. The increase in pH is preferably 1.9 or less. The increase in pH is more preferably 1.7 or less in order to further improve the ability to remove impurities. The lower limit of the increase in pH is typically, but not limited to, 0.01.
Specifically, the increase in pH can be determined from the formula below, and more specifically can be measured by the method described in the examples. In the present invention, the measurement of the increase in pH should be performed on the fresh affinity chromatography carrier. The fresh affinity chromatography carrier means the carrier unused before. For example, the fresh carrier for use in antibody purification means the carrier in the original state before use in antibody purification.

$$(\text{Increase in pH}) = (\text{loaded pH}) - (\text{initial pH})$$

In the above formula, "initial pH" means the pH value in the column at the time when the pH in the column reaches equilibrium after the 2 M NaCl-containing 20 mM sodium phosphate buffer with a pH of 7.5 is allowed to flow through the column.
In the above formula, "loaded pH" means the pH value at the time when the pH in the column reaches the highest level after a process that includes measuring the initial pH, then allowing the column to stand in a thermostat at 40°C for 7 days, and then allowing the 2 M NaCl-containing 20 mM sodium phosphate buffer with a pH of 7.5 to flow again through the column.
The ring-opened epoxy groups formed by subjecting the epoxy groups to the ring-opening are preferably represented by formula (1) in order to prevent nonspecific adsorption of impurities and prevent the ligand from decreasing in activity during storage.

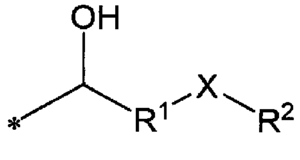
(1)

In formula (1), $R^1$ represents a divalent organic group of 1 to 6 carbon atoms, $R^2$ represents a hydrogen atom or a monovalent organic group of 1 to 10 carbon atoms, X represents a thio group (>S), a sulfinyl group (>S=O), a sulfonyl group ($>S(=O)_2$), an imino group (>NH), or an oxy group (>O), and * represents the bonding position.
$R^1$ represents a divalent organic group of 1 to 6 carbon atoms. This organic group preferably has 1 to 4 carbon atoms, more preferably 1 or 2 carbon atoms.
The divalent organic group may be linear or branched. The divalent organic group may be a divalent hydrocarbon group or a divalent hydrocarbon group having, between carbon atoms, one or more selected from the group consisting of an ether bond, an imino group, and an ester bond. The divalent organic group is preferably a divalent hydrocarbon group.

The divalent hydrocarbon group is preferably a divalent aliphatic hydrocarbon group, more preferably an alkanediyl group.

Examples of the alkanediyl group include methane-1,1-diyl, ethane-1,1-diyl, ethane-1,2-diyl, propane-1,1-diyl, propane-1,2-diyl, propane-1,3-diyl, propane-2,2-diyl, butane-1,4-diyl, pentane-1,5-diyl, and hexane-1,6-diyl.

$R^2$ represents a hydrogen atom or a monovalent organic group of 1 to 10 carbon atoms, preferably a monovalent organic group of 1 to 10 carbon atoms. In view of anti-fouling properties and the dynamic binding capacity obtained when the ligand is coupled, such an organic group is preferably an organic group represented by formula (2) or (3) below, more preferably an organic group represented by formula (2).

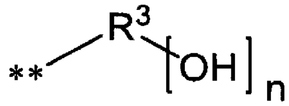

(2)

In formula (2), $R^3$ represents a divalent or trivalent organic group of 1 to 10 carbon atoms, n represents 1 or 2, and ** represents the position of bonding with X in formula (1).

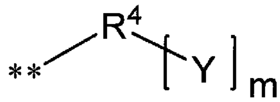

(3)

In formula (3), $R^4$ represents a divalent or trivalent organic group of 1 to 10 carbon atoms, Y represents an acidic group or an amino group, m represents 1 or 2, and ** represents the position of bonding with X in formula (1).

In view of, for example, the dynamic binding capacity obtained when the ligand is coupled, the divalent or trivalent organic group represented by $R^3$ in formula (2) or $R^4$ in formula (3) preferably has 1 to 8 carbon atoms, more preferably 1 to 6 carbon atoms, even more preferably 2 to 4 carbon atoms.

The divalent organic group may be a divalent hydrocarbon group, which may be linear or branched. The divalent hydrocarbon group is preferably a divalent aliphatic hydrocarbon group, more preferably an alkanediyl group. In view of, for example, the dynamic binding capacity obtained when the ligand is coupled, the alkanediyl group preferably has 1 to 8 carbon atoms, more preferably 1 to 6 carbon atoms, even more preferably 2 to 4 carbon atoms.

Examples of the alkanediyl group include methane-1,1-diyl, ethane-1,1-diyl, ethane-1,2-diyl, propane-1,1-diyl, propane-1,2-diyl, propane-1,3-diyl, and propane-2,2-diyl.

The trivalent organic group may be a trivalent hydrocarbon group, which may be linear or branched. The trivalent hydrocarbon group is preferably a trivalent aliphatic hydrocarbon group, more preferably an alkanetriyl group. In view of anti-fouling properties and the dynamic binding capacity obtained when the ligand is coupled, the alkanetriyl group preferably has 1 to 8 carbon atoms, more preferably 1 to 6 carbon atoms, even more preferably 2 to 4 carbon atoms.

Examples of the alkanetriyl group include methane-1,1,1-triyl, ethane-1,1,2-triyl, propane-1,2,3-triyl, and propane-1,2,2-triyl.

In formula (2), n represents 1 or 2, preferably 2.

In formula (3), Y represents an acidic group or an amino group, preferably an acidic group. The acidic group may be, for example, a carboxy group, a phosphate group, a sulfo group, or a salt thereof. The salt may be, for example, an alkali metal salt such as a sodium or potassium salt, an alkaline earth metal salt such as a magnesium or calcium salt, an ammonium salt, or an organic ammonium salt.

In formula (3), m represents 1 or 2, preferably 1.

X represents a thio group (>S), a sulfinyl group (>S=O), a sulfonyl group (>S $(=O)_2$), an imino group (>NH), or an oxy group (>O) , more preferably a thio group (>S), a sulfinyl group (>S=O) , or an oxy group (>O), even more preferably a thio group (>S) or a sulfinyl group (>S=O), further more preferably a sulfinyl group (>S=O).

A ring-opening method of the epoxy groups in the solid support may include, for example, stirring the solid support in an aqueous solvent in the presence of an acid or an alkali. The stirring may be performed with heating or at room temperature.

A blocking agent for ring-opening of epoxy groups may also be used, such as a hydrophilic group-containing mercapto compound such as mercaptoethanol, thioglycerol, mercaptoacetic acid or a salt thereof, mercaptosuccinic acid or a salt thereof, mercaptoethanesulfonic acid or a salt thereof, or mercaptopropanesulfonic acid or a salt thereof; a hydrophilic group-containing amine compound such as monoethanolamine or glucosamine; or a polyalcohol such as diethylene glycol or glycerin. In this case, examples of the hydrophilic group include a hydroxy group, an amino group, and acidic groups such as a carboxy group, a phosphate group, a sulfo group, and a salt thereof. Examples of the salt include alkali metal salts such as sodium salts and potassium salts, alkaline earth metal salts such as magnesium salts

and calcium salts, ammonium salts, and organic ammonium salts.

The acid or alkali and the blocking agent are each generally used in an amount of 0.1 to 40 molar equivalents per 1 mole of residual epoxy groups. Residual epoxy groups can cause multi-point bonding with a ligand to reduce the ligand activity during storage. Therefore, blocking is preferably performed using an excess amount of 2 to 40 molar equivalents.

The ring-opening reaction time is generally, but not limited to, about 0.5 to about 72 hours, preferably 1 to 48 hours . The reaction temperature may be appropriately selected from temperatures not higher than the boiling point of the solvent. In general, the reaction temperature is from about 2 to about 100°C.

If necessary, the blocking may also be performed in the presence of a basic catalyst. Examples of such a basic catalyst include triethylamine, N,N-dimethyl-4-aminopyridine, and diisopropylethylamine, which may be used alone or in combination of two or more.

The ring-opened epoxy groups are preferably those obtained by subjecting the epoxy groups in the solid support to a ring-opening using a mercapto compound and/or a polyalcohol. Such ring-opened epoxy groups have the advantage that they are less likely to cause nonspecific adsorption than ring-opened groups formed using an amino group-containing blocking agent and can provide high dynamic binding capacity.

When a mercapto compound is used as the blocking agent, the thio group can be oxidized to a sulfinyl group using an oxidizing agent, so that the hydrophilicity can be further improved.

The oxidizing agent can be roughly classified into organic and inorganic oxidizing agents. Examples of the organic oxidizing agent include peracetic acid, perbenzoic acid, and m-chloroperbenzoic acid. On the other hand, examples of the inorganic oxidizing agent include hydrogen peroxide, chromic acid, and permanganates. These oxidizing agents may be used alone or in combination of two or more.

The oxidizing agent is generally used in a total amount of about 0.1 to about 10 molar equivalents, preferably 0.5 to 3 molar equivalents, per 1 mole of thio groups.

The oxidation reaction is preferably performed in the presence of a solvent. Examples of such a solvent include water, amide solvents such as dimethylformamide and dimethylacetamide, and alcohol solvents such as methanol and ethanol. These solvents may be used alone or in combination of two or more.

The solvent is generally used in a total amount of about 1 to about 50 times by mass, preferably 5 to 15 times by mass, with respect to the solid support as a raw material.

The oxidation reaction time is generally, but not limited to, about 1 to about 72 hours. The reaction temperature may be appropriately selected from temperatures not higher than the boiling point of the solvent. In general, the reaction temperature is from about 1 to about 90°C.

(Ligand)

The affinity chromatography carrier of the present invention contains a ligand coupled to the solid support.

The ligand may be of any type having a suitable level of affinity for target materials. Examples of the ligand include, but are not limited to, proteins such as protein A, protein G, protein L, Fc-binding proteins, and avidin, peptides such as insulin, antibodies such as monoclonal antibodies, enzymes, hormones, DNA, RNA, saccharides such as heparin, Lewis X, and ganglioside, and low-molecular-weight compounds such as iminodiacetic acid, synthetic dyes, 2-aminophenylboronic acid, 4-aminobenzamidine, glutathione, biotin, and derivatives thereof. The ligands listed above may be used as they are, or their recombinant forms or their fragments obtained by enzyme treatment or the like may also be used. The ligand may also be an artificially synthesized peptide or peptide derivative.

Among the above ligands, one of the ligands suitable for isolation or purification of immunoglobulins is an immunoglobulin-binding protein.

The immunoglobulin-binding protein is preferably one or more selected from the group consisting of protein A, protein G, protein L, Fc-binding protein, and functional variants thereof. In particular, protein A, protein G, and functional variants thereof are preferred, and protein A or a functional variant thereof is more preferred.

In the present invention, the term "protein" refers to any molecule having a peptide structural unit, which is a concept encompassing, for example, partial fragments of natural proteins and natural protein variants obtained by artificially modifying the amino acid sequences of natural proteins. The term "immunoglobulin-binding domain" refers to a functional unit of polypeptide having immunoglobulin-binding activity by itself. The term "immunoglobulin-binding protein" refers to an immunoglobulin-binding domain-containing protein having specific affinity for immunoglobulin. The term "immunoglobulin-binding" refers to having the ability to bind to a region other than the complementarity determining region (CDR) in an immunoglobulin molecule, specifically, the Fc fragment. In the present invention, the term "ligand" used in connection with affinity chromatography refers to a molecule capable of binding to a target material for affinity chromatography.

In a method of coupling the ligand to the solid support, the epoxy groups contained in the solid support are preferably used without any modification as sites for coupling to the ligand, so that the process will be simple. Another

method may include using, for example, tosyl groups to activate alcoholic hydroxyl groups produced by the ring-opening of the epoxy groups contained in the solid support and then coupling the ligand to the solid support, or may include further extending a linker from the epoxy groups contained in the solid support or from the groups produced by the ring-opening of the epoxy groups and then coupling the ligand to the solid support via the linker. The linker is preferably a diglycidyl ether compound such as diglycidyloxyalkane or polyalkylene glycol diglycidyl ether.

Methods known to those skilled in the art may be used for ligand coupling although the ligand coupling conditions depend on the epoxy group content of the solid support before the ligand coupling and the type of the ligand. When the ligand is a protein, the N-terminal amino group of the protein and lysine and cysteine residues in the protein can serve as reactive sites to epoxy. A protein can be coupled as the ligand, for example, by a process that includes using an aqueous solution of a buffer with an isoelectric point close to that of the protein, optionally adding a salt such as sodium chloride or sodium sulfate to the solution, and allowing the protein and the solid support to react in the solution at 0 to 40°C for 1 to 48 hours while mixing them.

The amount of coupling of the ligand can be appropriately controlled depending on the type of the ligand and the type of the target material. For example, an immunoglobulin-binding protein such as protein A is preferably coupled as the ligand in an amount of 10 to 200 mg, more preferably 25 to 100 mg, per 1 g of the solid support. When an immunoglobulin-binding protein such as protein A is coupled as the ligand, the ligand coupled in an amount of 10 mg or more per 1 g of the solid support can provide high dynamic binding capacity, and the ligand coupled in an amount of 200 mg or less can make it possible to elute a bound antibody using a particularly suitable amount of the eluate.

(Particle size and specific surface area)

When the solid support constituting the affinity chromatography carrier of the present invention is in the form of porous particles, the porous particles generally have a particle size of 35 to 100 μm, preferably 40 to 85 μm. The porous particles with a particle size of 35 μm or more have good pressure properties. The porous particles with a particle size of 100 μm or less can form a filler with high dynamic binding capacity.

The particle size of the porous particles can be controlled by the conditions of the polymerization. In the present invention, the term "particle size" means the volume average particle size determined by a laser diffraction scattering particle size distribution analyzer (LS 13 320 manufactured by Beckman Coulter, Inc.).

When the solid support constituting the affinity chromatography carrier of the present invention is in the form of porous particles, the porous particles preferably have a specific surface area of 70 $m^2/g$ or more, more preferably 90 $m^2/g$ or more, in terms of the pore size range of 10 nm to 5,000 nm, when pores with diameters in the range of 10 to 5,000 nm are measured. When the specific surface area falls within the above range, the resulting affinity chromatography carrier can have high dynamic binding capacity for target materials. In the present invention, the "specific surface area" is the value obtained by measuring the surface area of pores with diameters of 10 to 5,000 nm using a mercury porosimeter (AutoPore IV 9520 manufactured by SHIMADZU CORPORATION) and dividing the surface area by the dry weight of the particles.

The affinity chromatography carrier of the present invention has a high ability to remove impurities and also has high storage stability. The affinity chromatography carrier of the present invention is particularly useful for purification of proteins such as antibodies.

[Affinity chromatography column]

The affinity chromatography column of the present invention includes a column vessel and the affinity chromatography carrier of the present invention with which the column vessel is packed.

[Method for purifying target material]

The method for purifying a target material according to the present invention is characterized by using the affinity chromatography carrier of the present invention.

The purification may be similar to a conventional method, except that the affinity chromatography carrier of the present invention is used. The purification method of the present invention may include, for example, the steps of: preparing a composition containing a target material; allowing the composition to flow through the chromatography column of the present invention to adsorb the target material to the carrier; and eluting the adsorbed target material from the carrier.

The purification method of the present invention is suitable for purification of proteins and particularly suitable for purification of immunoglobulins.

Examples

Hereinafter, the present invention will be more specifically described with reference to examples. It will be understood that the examples are not intended to limit the present invention.

(Example 1) (Reference)

(1) To 360 g of pure water was added 3.58 g of polyvinyl alcohol (PVA-217 manufactured by KURARAY CO., LTD.) and dissolved by heating and stirring. After the polyvinyl alcohol solution was cooled, 0.36 g of sodium dodecyl sulfate (manufactured by Wako Pure Chemical Industries, Ltd.), 0.36 g of sodium sulfate (manufactured by Wako Pure Chemical Industries, Ltd.), and 0.18 g of sodium nitrite (manufactured by Wako Pure Chemical Industries, Ltd.) were added to the solution and stirred to form an aqueous solution S.
On the other hand, a monomer composition containing 12.00 g of glycidyl methacrylate (manufactured by MITSUBISHI RAYON CO., LTD.) and 1.33 g of divinylbenzene (manufactured by Nippon Steel Chemical Co., Ltd.) was dissolved in 24.43 g of diisobutyl ketone (manufactured by Mitsui Chemicals, Inc.) to form a monomer solution.
Subsequently, the entire aqueous solution S was added to a 500 mL separable flask, to which a thermometer, a stirring blade, and a condenser tube were attached. The flask was then set in a warm water bath, and the solution S started to be stirred under a nitrogen atmosphere. The entire monomer solution was then added to the separable flask. When the inner temperature was raised to 85°C by heating with the warm water bath, 0.53 g of 2,2'-azoisobutyronitrile (manufactured by Wako Pure Chemical Industries, Ltd.) was added, and the temperature was maintained at 86°C.
(2) Subsequently, stirring was performed for 3 hours while the temperature was maintained at 86°C. Subsequently, the reaction liquid was cooled and then subjected to filtration. The resulting particles were washed with pure water and ethanol. The washed particles were dispersed in pure water and then subjected to decantation three times, so that small particles were removed. Subsequently, the particles were dispersed at a concentration of 10% by mass in pure water to form a dispersion of porous particles.
(3) A protein A dispersion was obtained by dispersing 0.15 g of modified protein A (rSPA manufactured by Repligen Corporation) in 40 mL of a 1.2 M sodium sulfate/0.1 M sodium phosphate buffer (pH 6.6). The dispersion of porous particles obtained in the step (2) of Example 1 (corresponding to 1 g in terms of particle dry weight) was added to the protein A dispersion. The resulting dispersion was stirred by shaking at 25°C for 5 hours so that the protein A was immobilized on the particles.
(4) The resulting particles with immobilized protein A were washed with a 0.1 M sodium phosphate buffer (pH 6.6) and then dispersed in 40 mL of a 1.0M sulfuric acid aqueous solution (manufactured by Wako Pure Chemical Industries, Ltd.). The resulting dispersion was stirred by shaking at 40°C for 4 hours so that the unreacted epoxy groups were subjected to the ring-opening.

Subsequently, the particles were washed with a 0.1 M sodium phosphate buffer (pH 6.6), a 0.1 M sodium hydroxide aqueous solution, and a 0.1 M sodium citrate buffer (pH 3.2) to give filler 1 for affinity chromatography.

(Example 2)

A dispersion of porous particles was obtained by the same operations as in the steps (1) and (2) of Example 1, except that the monomer solution was prepared by dissolving a monomer composition containing 9.20 g of glycidyl methacrylate (manufactured by MITSUBISHI RAYON CO., LTD.), 0.58 g of 1-ethyl-4-vinylbenzene (manufactured by ChemSampCo), and 1.73 g of divinylbenzene (manufactured by Nippon Steel Chemical Co., Ltd.) in a mixed solution of 19.59 g of diisobutyl ketone (manufactured by Mitsui Chemicals, Inc.) and 6.05 g of anisole (manufactured by Wako Pure Chemical Industries, Ltd.) in the step (1) of Example 1.
Subsequently, protein A was immobilized by the same operation as in the step (3) of Example 1, so that particles with immobilized protein A were obtained.
Subsequently, the resulting particles with immobilized protein A were dispersed in 40 mL of 1.0 M 2-mercaptoethanol (manufactured by Wako Pure Chemical Industries, Ltd.)/0.1 M sodium sulfate (pH 8.3) and then stirred by shaking at 25°C for 17 hours so that the unreacted epoxy groups were subjected to the ring-opening. The resulting particles with immobilized protein A and ring-opened unreacted epoxy groups were then washed with a 0.1 M sodium phosphate buffer (pH 6.6), a 0.1 M sodium hydroxide aqueous solution, and a 0.1 M sodium citrate buffer (pH 3.2) to give filler 2 for affinity chromatography.

(Example 3)

A dispersion of porous particles was obtained by the same operations as in the steps (1) and (2) of Example 1, except that the monomer solution was prepared by dissolving a monomer composition containing 8.18 g of glycidyl methacrylate (manufactured by MITSUBISHI RAYON CO., LTD.), 1.36 g of N-(2-hydroxyethyl)acrylamide (manufactured by Tokyo Chemical Industry Co., Ltd.), and 4.09 g of trimethylolpropane trimethacrylate (manufactured by Sartomer) in a mixed solution of 21.97 g of 4-methyl-2-pentanol (manufactured by Wako Pure Chemical Industries, Ltd.) and 2.95 g of diethyl carbonate (manufactured by Wako Pure Chemical Industries, Ltd.) in the step (1) of Example 1.
Subsequently, protein A was immobilized by the same operation as in the step (3) of Example 1, so that particles with immobilized protein A were obtained.
Subsequently, the resulting particles (PA-1) with immobilized protein A were dispersed in 40 mL of 1.0 M 1-thioglycerol (manufactured by ASAHI CHEMICAL Co., Ltd.)/0.1 M sodium sulfate (pH 8.3) and then stirred by shaking at 25°C for 17 hours so that the unreacted epoxy groups were subjected to ring-opening. The resulting particles (PA-2) with immobilized protein A and ring-opened unreacted epoxy groups were then washed with a 0.1 M sodium phosphate buffer (pH 6.6), a 0.1 M sodium hydroxide aqueous solution, and a 0.1 M sodium citrate buffer (pH 3.2) to give filler 3 for affinity chromatography.

(Example 4)

Particles (PA-2) with immobilized protein A and ring-opened unreacted epoxy groups, which were obtained by the same operations as in Example 3, were dispersed at a concentration of 10% by mass in pure water. To the dispersion was added 0.48 g of 30% hydrogen peroxide water and stirred by shaking at 25°C for 24 hours. The resulting particles were then washed with a 0.1 M sodium phosphate buffer (pH 6.6), a 0.1 M sodium hydroxide aqueous solution, and a 0.1 M sodium citrate buffer (pH 3.2) to give filler 4 for affinity chromatography.

(Example 5)

Particles (PA-1) with immobilized protein A, which were produced by the same operations as in Example 3, were dispersed in 40 mL of 1.0 M 1-thioglycerol/0.1 M sodium sulfate (pH 8.3) and then stirred by shaking at 25°C for 40 hours so that the unreacted epoxy groups were subjected to ring-opening. The resulting particles with immobilized protein A and ring-opened unreacted epoxy groups were then washed with a 0.1 M sodium phosphate buffer (pH 6.6), a 0.1 M sodium hydroxide aqueous solution, and a 0.1 M sodium citrate buffer (pH 3.2) to give filler 5 for affinity chromatography.

(Example 6)

A dispersion of porous particles was obtained by the same operations as in the steps (1) and (2) of Example 1, except that the monomer solution was prepared by dissolving a monomer composition containing 5.26 g of glycidyl methacrylate (manufactured by MITSUBISHI RAYON CO., LTD.), 2.63 g of glycerol monomethacrylate (manufactured by NOF CORPORATION), and 2.63 g of divinylbenzene (manufactured by Nippon Steel Chemical Co., Ltd.) in 25.00 g of 2-octanone (manufactured by Toyo Gosei Co., Ltd.) in the step (1) of Example 1.
Subsequently, protein A was immobilized by the same operation as in the step (3) of Example 1, so that particles with immobilized protein A were obtained.
Subsequently, the resulting particles with immobilized protein A were dispersed in 40 mL of 1.0 M sodium 2-mercaptopropanesulfonate (manufactured by Tokyo Chemical Industry Co., Ltd.)/0.1 M sodium sulfate (pH 8.3) and then stirred by shaking at 25°C for 17 hours so that the unreacted epoxy groups were subjected to ring-opening. The resulting particles with immobilized protein A and ring-opened unreacted epoxy groups were then washed with a 0.1 M sodium phosphate buffer (pH 6.6), a 0.1 M sodium hydroxide aqueous solution, and a 0.1 M sodium citrate buffer (pH 3.2) to give filler 6 for affinity chromatography.

(Example 7) (Reference)

A dispersion of porous particles was obtained by the same operations as in the steps (1) and (2) of Example 1, except that the monomer solution was prepared by dissolving a monomer composition containing 4.77 g of 4-hydroxybutyl acrylate glycidyl ether (manufactured by Nippon Kasei Chemical Company Limited) and 8.86 g of glycerin dimethacrylate (manufactured by Shin Nakamura Chemical Co., Ltd.) in a mixed solution of 21.52 g of acetophenone (manufactured by Inoue Perfumery MFG. Co., Ltd.) and 7.35 g of 2-octanone (manufactured by Toyo Gosei Co., Ltd.) in the step (1) of Example 1.

Subsequently, the same operations as in Example 3 were performed to immobilize protein A, subjecting the unreacted epoxy groups to ring-opening, and wash the particles, so that filler 7 for affinity chromatography was obtained.

(Example 8) (Reference)

A dispersion of porous particles was obtained by the same operations as in the steps (1) and (2) of Example 1, except that the monomer solution was prepared by dissolving a monomer composition containing 3.43 g of vinylbenzyl glycidyl ether (manufactured by Toray Fine Chemicals Co., Ltd.), 4.11 g of glycerol monomethacrylate (manufactured by NOF CORPORATION), and 6.17 g of ethylene glycol dimethacrylate (manufactured by Shin Nakamura Chemical Co., Ltd.) in a mixed solution of 21.52 g of acetophenone (manufactured by Inoue Perfumery MFG. Co., Ltd.) and 7.35 g of 2-octanone (manufactured by Toyo Gosei Co., Ltd.) in the step (1) of Example 1.

Subsequently, the same operations as in Example 3 were performed to immobilize protein A, subjecting the unreacted epoxy groups to ring-opening, and wash the particles, so that filler 8 for affinity chromatography was obtained.

(Comparative Example 1)

Filler 9 for affinity chromatography was obtained by the same operations as in Example 3, except that ring-opening of the unreacted epoxy groups was not performed after the immobilization of protein A.

(Comparative Example 2)

A dispersion of porous particles was obtained by the same operations as in the steps (1) and (2) of Example 1, except that the monomer solution was prepared by dissolving a monomer composition containing 1.41 g of 3,4-epoxycyclohexylmethyl methacrylate (manufactured by Daicel Corporation), 8.45 g of glycerol monomethacrylate (manufactured by NOF CORPORATION), and 4.23 g of trimethylolpropane trimethacrylate (manufactured by Sartomer) in a mixed solution of 21.50 g of acetophenone (manufactured by Inoue Perfumery MFG. Co., Ltd.) and 7.34 g of 2-octanone (manufactured by Toyo Gosei Co., Ltd.) in the step (1) of Example 1.

Subsequently, protein A was immobilized by the same operation as in the step (3) of Example 1, so that particles with immobilized protein A were obtained.

Subsequently, the resulting particles with immobilized protein A were dispersed in 40 mL of 1.0 M 1-thioglycerol (manufactured by ASAHI CHEMICAL Co., Ltd.) /0.1 M sodium sulfate (pH 8.3) and then stirred by shaking at 25°C for 4 hours so that the unreacted epoxy groups were subjected to ring-opening. The resulting particles were then washed with a 0.1 M sodium phosphate buffer (pH 6.6), a 0.1 M sodium hydroxide aqueous solution, and a 0.1 M sodium citrate buffer (pH 3.2) to give filler 10 for affinity chromatography.

(Comparative Example 3)

Filler 11 for affinity chromatography was obtained by the same operations as in Comparative Example 2, except that the particles with immobilized protein A were stirred by shaking for 17 hours instead of 4 hours after the particles with immobilized protein A were dispersed in 40 mL of 1.0 M 1-thioglycerol (manufactured by ASAHI CHEMICAL Co., Ltd.)/0.1 M sodium sulfate (pH 8.3).

(Comparative Example 4)

A dispersion of porous particles was obtained by the same operations as in the steps (1) and (2) of Example 1, except that the monomer solution was prepared by dissolving a monomer composition containing 1.39 g of glycidyl methacrylate (manufactured by MITSUBISHI RAYON CO., LTD.), 2.79 g of glycerol monomethacrylate (manufactured by NOF CORPORATION), and 9.76 g of glycerin dimethacrylate (manufactured by Shin Nakamura Chemical Co., Ltd.) in a mixed solution of 21.50 g of acetophenone (manufactured by Inoue Perfumery MFG. Co., Ltd.) and 7.34 g of 2-octanone (manufactured by Toyo Gosei Co., Ltd.) in the step (1) of Example 1.

Subsequently, the same operations as in Example 3 were performed to immobilize protein A, subjecting the unreacted epoxy groups to ring-opening, and wash the particles, so that filler 12 for affinity chromatography was obtained.

Test Example 1 (Determination of epoxy group content)

The epoxy group content of the porous particles obtained in each of Examples 1 to 8 and Comparative Examples 1 to 4 was determined before the ligand coupling.

Specifically, excess hydrochloric acid was added to the porous particles suspended in a calcium chloride

aqueous solution to cause the addition reaction of hydrochloric acid thereto, so that the epoxy groups in the porous particles were subjected to ring-opening. After the residual hydrochloric acid was neutralized with an excess of a sodium hydroxide aqueous solution, a phenolphthalein solution was added thereto, and then the epoxy group content was determined by back titration of the residual sodium hydroxide with hydrochloric acid. The end point of the titration was the point where the red color of phenolphthalein disappeared. Table 1 shows the results.

Test Example 2 (Evaluation of the amount of residual epoxy groups (measurement of increase in pH))

Tricorn 5/50 Column manufactured by GE Healthcare was packed with the filler (with a height of 5 cm) for affinity chromatography obtained in each of Examples 1 to 8 and Comparative Example 1 to 4. The column was then connected to AKTAprime Plus manufactured by GE Healthcare. While the pH was monitored, a 2 M NaCl-containing 20 mM sodium phosphate buffer with a pH of 7.5 was allowed to flow at a rate of 0.2 mL/minute through the column. When the pH in the column reached equilibrium, the resulting pH value was recorded as the initial pH. The column purged with the buffer was detached from AKTAprime Plus and then allowed to stand in a thermostat at 40°C for 7 days.

Subsequently, the column was connected again to AKTAprime Plus. While the pH was monitored, the buffer was allowed to flow at a rate of 0.2 mL/minute through the column. When the pH in the column reached the highest value, the resulting pH value was recorded as the loaded pH. An increase in pH was calculated by subtracting the initial pH from the loaded pH. The initial pH and the loaded pH were measured in a thermostatic chamber at 23 ± 1°C. Table 1 shows the results.

Test Example 3 (Determination of the amount of coupled protein A)

The amount of protein A coupled as a ligand to the filler for affinity chromatography obtained in each of Examples 1 to 8 and Comparative Examples 1 to 4 was determined using bicinchoninic acid (BCA) reagents. Specifically, 1 mg (on a solid basis) of the filler was collected in a test tube and then subjected to quantification using BCA Protein Assay Kit from Thermo Fisher Scientific. The reaction was performed by inversion mixing at 37°C for 30 minutes. A calibration curve was prepared using protein A, which was the same as that coupled to the carrier. Table 1 shows the results.

Test Example 4 (Measurement of DBC and evaluation of storage stability)

Using AKTAprime Plus manufactured by GE Healthcare, the dynamic binding capacity (DBC) of the filler of each of the examples and the comparative examples was measured at a linear flow rate of 300 cm/hr for a protein (human IgG antibody HGG-1000 manufactured by Equitech-Bio, Inc.). The column used has a volume of 4 mL (5 mmφ x 200 mm long), and the solution used was obtained by dissolving 5 mg/mL of the protein in a 20 mM sodium phosphate/150 mM sodium chloride aqueous solution (pH 7.5). The DBC was determined from the column packing volume and the amount of the captured protein at 10% breakthrough for the elution peak. Table 1 shows the results.

After the measurement of DBC, the column was purged with a 2 M NaCl-containing 20 mM sodium phosphate buffer with a pH of 7.5 as in Test Example 2 and then allowed to stand at 40°C for 7 days without any modifications. Subsequently, the DBC was measured under the same conditions as those described above, so that the loaded DBC was determined. The loaded DBC/the initial DBC x 100 was calculated and used as the DBC retention ratio to evaluate the storage stability of the filler for affinity chromatography. The initial DBC and the loaded DBC were measured in a thermostatic chamber at 23 ± 1°C. Table 1 shows the results.

Test Example 5 (Quantification of HCP)

A packed column was prepared by packing a column vessel (Tricorn 10/50 Column manufactured by GE Healthcare) with the filler (with a height of about 5 cm) obtained in each of the examples and the comparative examples. Each resulting packed column was connected to AKTAprime Plus manufactured by GE Healthcare. A five-column volume (five times the volume of the column) of a 20 mM sodium phosphate buffer (pH 7.5) was then allowed to flow at a rate of 1 mL/minute through the column until equilibrium was reached.

Subsequently, a CHO cell culture supernatant containing a monoclonal antibody, Trastuzumab was allowed to flow at a rate of 1 mL/minute with a load of about 23 mg antibody/mL carrier through the column.

Subsequently, five-column volumes of a 20 mM sodium phosphate buffer (pH 7.5), a 20 mM sodium phosphate/1 M sodium chloride buffer (pH 7.5), and a 20 mM sodium phosphate buffer (pH 7.5) were sequentially allowed to flow at a rate of 1 mL/minute through the column.

A 50 mM sodium citrate buffer (pH 3.2) was then allowed to flow at a rate of 1 mL/minute through the column, so that the monoclonal antibody captured in the column was eluted. Eluted fractions with Abs. 280 > 100 mAu were collected.

The concentration (mg/mL) of the antibody in the collected fractions was measured using a spectrophotometer. The concentration (ng/mL) of host cell protein (HCP) in the collected fractions was also measured using CHO HCP ELISA Kit 3G manufactured by Cygnus Technologies Inc. The amount of HCP per unit amount of antibody was calculated by dividing the HCP concentration by the antibody concentration. Table 1 shows the results.

[Table 1]

| | | Monomer composition (parts by mass) | | | Epoxy group content | Particle size | Amount of coupled protein A | Ring-opened epoxy group (Ring-opening conditions) | Oxidizing agent | pH | DBC | HCP | Storage stability |
|---|---|---|---|---|---|---|---|---|---|---|---|---|---|
| | | M-1 | M-2 | M-3 | mmol/g | μm | mg/g | | | Increase | mg/mL | ppm | % |
| Example | 1* | GMA 90 | DVB 10 | - | 4.43 | 78 | 83 | Sulfuric acid, 40°C×4 hr | Absent | 0.47 | 35.5 | 1203 | 94 |
| | 2 | GMA 80 | DVB 15 | EVB 5 | 3.94 | 61 | 53 | ME, 25°C×17hr | Absent | 1.81 | 39.7 | 1633 | 97 |
| | 3 | GMA 60 | TMP 30 | HEAA 10 | 2.96 | 63 | 65 | TG,25°C×17hr | Absent | 1.74 | 40.1 | 1102 | 96 |
| | 4 | GMA 60 | TMP 30 | HEAA 10 | 2.96 | 63 | 65 | TG, 25°C×17hr | $H_2O_2$ | 1.69 | 38.4 | 877 | 98 |
| | 5 | GMA 60 | TMP 30 | HEAA 10 | 2.96 | 63 | 65 | TG, 25°C×40hr | Absent | 1.40 | 38.2 | 790 | 100 |
| | 6 | GMA 50 | DVB 25 | GLM 25 | 2.40 | 59 | 57 | MPSA, 25°C×17hr | Absent | 1.33 | 40.2 | 1828 | 96 |
| | 7* | HBAGE 35 | GLDM 65 | - | 1.22 | 47 | 59 | TG, 25°C×17hr | Absent | 1.21 | 36.0 | 1005 | 85 |
| | 8* | VBGE 25 | EDMA 45 | GLM 30 | 1.29 | 52 | 61 | TG, 25°C×17hr | Absent | 1.28 | 34.9 | 1569 | 82 |
| Comparative Example | 1 | GMA 60 | TMP 30 | HEAA 10 | 2.96 | 63 | 65 | Absent | Absent | 3.55 | 33.2 | 5982 | 51 |
| | 2 | METHB 10 | TMP 30 | GLM 60 | 0.35 | 55 | 66 | TG, 25°C×4hr | Absent | 2.87 | 36.4 | 4714 | 63 |
| | 3 | METHB 10 | TMP 30 | GLM 60 | 0.35 | 55 | 66 | TG, 25°C×17hr | Absent | 1.42 | 34.1 | 3123 | 81 |
| | 4 | GMA 10 | GLDM 70 | GLM 20 | 0.38 | 48 | 52 | TG, 25°C×17hr | Absent | 1.47 | 38.7 | 3268 | 80 |

* Reference Example

In Table 1, each variable represents the following.

(M-1) GMA: Glycidyl methacrylate, HBAGE: 4-Hydroxybutyl acrylate glycidyl ether, VBGE: (4-Vinylbenzyl) glycidyl ether, METHB: 3,4-Epoxycyclohexylmethyl methacrylate
(M-2) DVB: Divinylbenzene, TMP: Trimethylolpropane trimethacrylate, GLDM: Glycerin dimethacrylate, EDMA: Ethylene glycol dimethacrylate
(M-3) EVB: 1-Ethyl-4-vinylbenzene, HEAA: N-(2-hydroxyethyl)methacrylamide, GLM: Glycerol menomethacrylate
(Compounds used for ring-opening) ME: 2-Mercaptoethanol, TG: 1-Thioglycerol, MPSA: Sodium 2-mercaptopropanesulfonate

## Claims

**1.** An affinity chromatography carrier, the carrier comprising:

a solid support comprising a copolymer comprising (M-1) 40 parts by mass or more to 80 parts by mass or less of a structural unit derived from an epoxy group-containing monovinyl monomer with respect to 100 parts by mass of all structural units and (M-2) 15 to 60 parts by mass of a structural unit derived from a polyvinyl monomer with respect to 100 parts by mass of all structural units, wherein the copolymer further comprises (M-3) 5 to 25 parts by mass of a structural unit derived from an epoxy-free monovinyl monomer with respect to 100 parts by mass of all structural units, wherein the epoxy-free monovinyl monomer is selected from the group consisting of ethylvinylbenzene, glycerol mono(meth)acrylate and hydroxyethyl(meth)acrylamide;
ring-opened epoxy groups obtained by subjecting the epoxy groups to the ring-opening; and
a ligand coupled to the solid support,
the affinity chromatography carrier being such that after a certain column vessel is packed with the carrier and then purged with a 2 M NaCl-containing 20 mM sodium phosphate buffer with a pH of 7.5, allowing the column to stand at 40°C for 7 days increases pH in the column by at most 2, and
the ring-opened epoxy groups being represented by formula (1):

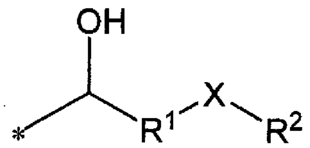
(1)

wherein $R^1$ represents a divalent organic group of 1 to 6 carbon atoms, $R^2$ represents a hydrogen atom or a monovalent organic group of 1 to 10 carbon atoms, X represents a thio group (>S), a sulfinyl group (>S=O), a sulfonyl group ($>S(=O)_2$), an imino group (>NH), or an oxy group (>O), and * represents a bonding position.

**2.** The affinity chromatography carrier according to claim 1, wherein $R^2$ is represented by formula (2):

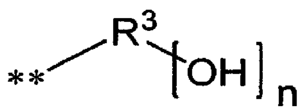
(2)

wherein $R^3$ represents a divalent or trivalent organic group of 1 to 10 carbon atoms, n represents 1 or 2, and ** represents a position of bonding with X in formula (1).

**3.** The affinity chromatography carrier according to claim 1 or 2, wherein X is a thio group (>S), a sulfinyl group (>S=O), or an oxy group (>O).

**4.** The affinity chromatography carrier according to any one of claims 1 to 3, wherein X is a thio group (>S), or a sulfinyl group (>S=O).

**5.** The affinity chromatography carrier according to any one of claims 1 to 4, wherein the ligand is an immunoglobulin-binding protein.

6. The affinity chromatography carrier according to claim 5, wherein the immunoglobulin-binding protein is one or more selected from the group consisting of protein A, protein G, protein L, Fc-binding protein, and functional variants thereof.

7. The affinity chromatography carrier according to any one of claims 1 to 6, wherein the solid support is in the form of a porous particle.

8. The affinity chromatography carrier according to claim 7, wherein the porous particle has a volume average particle size determined by a laser diffraction scattering particle size distribution analyzer of 35 to 100 μm.

9. An affinity chromatography column comprising: a column vessel; and the affinity chromatography carrier according to any one of claims 1 to 8 with which the column vessel is packed.

10. A method for purifying a target material, comprising using the affinity chromatography carrier according to any one of claims 1 to 8.

11. The method according to claim 10, wherein the target material is a target protein.

## Patentansprüche

1. Affinitätschromatographieträger, wobei der Träger umfasst:

einen festen Träger, der ein Copolymer umfasst, das (M-1) 40 Masseteile oder mehr bis 80 Masseteile oder weniger einer Struktureinheit, die von einem epoxygruppenhaltigen Monovinylmonomer abgeleitet ist, in Bezug auf 100 Masseteile aller Struktureinheiten und (M-2) 15 bis 60 Masseteile einer Struktureinheit, die von einem Polyvinylmonomer abgeleitet ist, in Bezug auf 100 Masseteile aller Struktureinheiten umfasst, wobei das Copolymer ferner (M-3) 5 bis 25 Masseteile einer Struktureinheit, die von einem epoxyfreien Monovinylmonomer abgeleitet ist, bezogen auf 100 Masseteile aller Struktureinheiten umfasst, wobei das epoxyfreie Monovinylmonomer ausgewählt ist aus der Gruppe bestehend aus Ethylvinylbenzol, Glycerinmono(meth)acrylat und Hydroxyethyl(meth)acrylamid;
ringgeöffnete Epoxygruppen, erhalten durch Unterziehen der Epoxygruppen der Ringöffnung; und
einen Liganden, der an den festen Träger gekoppelt ist,
wobei der Affinitätschromatographieträger so beschaffen ist, daß, nachdem ein bestimmtes Säulengefäß mit dem Träger gepackt und dann mit einem 2 M NaCl enthaltenden 20 mM Natriumphosphatpuffer mit einem pH-Wert von 7,5 gespült wurde, das Stehenlassen der Säule bei 40°C für 7 Tage den pH-Wert in der Säule um höchstens 2 erhöht, und
wobei die ringgeöffneten Epoxygruppen durch die Formel (1) dargestellt sind:

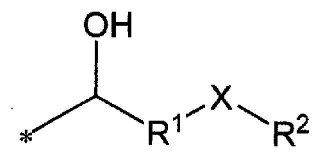
(1)

worin $R^1$ eine zweiwertige organische Gruppe mit 1 bis 6 Kohlenstoffatomen darstellt, $R^2$ ein Wasserstoffatom oder eine einwertige organische Gruppe mit 1 bis 10 Kohlenstoffatomen darstellt, X eine Thiogruppe (>S), eine Sulfinylgruppe (>S=O), eine Sulfonylgruppe (>S(=O)$_2$), eine Iminogruppe (>NH) oder eine Oxygruppe (>O) darstellt, und * eine Bindungsposition darstellt.

2. Affinitätschromatographieträger nach Anspruch 1, wobei $R^2$ durch die Formel (2) dargestellt ist:

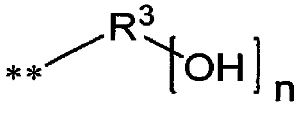
(2)

wobei $R^3$ eine zweiwertige oder dreiwertige organische Gruppe mit 1 bis 10 Kohlenstoffatomen darstellt, n 1 oder 2 darstellt, und ** eine Bindungsposition mit X in Formel (1) darstellt.

**3.** Affinitätschromatographieträger nach Anspruch 1 oder 2, wobei X eine Thiogruppe (>S), eine Sulfinylgruppe (>S=O) oder eine Oxygruppe (>O) ist.

**4.** Affinitätschromatographieträger nach einem der Ansprüche 1 bis 3, wobei X eine Thiogruppe (>S), oder eine Sulfinylgruppe (>S=O) ist.

**5.** Affinitätschromatographieträger nach einem der Ansprüche 1 bis 4, wobei der Ligand ein Immunglobulin-bindendes Protein ist.

**6.** Affinitätschromatographieträger nach Anspruch 5, wobei das Immunglobulin-bindende Protein eines oder mehrere ist, ausgewählt aus der Gruppe bestehend aus Protein A, Protein G, Protein L, Fc-bindendem Protein und funktionellen Varianten davon.

**7.** Affinitätschromatographieträger nach einem der Ansprüche 1 bis 6, wobei der feste Träger in Form eines porösen Teilchens vorliegt.

**8.** Affinitätschromatographieträger nach Anspruch 7, wobei das poröse Teilchen eine volumengemittelte Teilchengröße, die durch einen Laserbeugungsstreuungsteilchengrößenanalysator gemessen ist, von 35 bis 100 μm aufweist.

**9.** Affinitätschromatographiesäule, umfassend: ein Säulengefäß; und den Affinitätschromatographieträger nach einem der Ansprüche 1 bis 8, mit dem das Säulengefäß gepackt ist.

**10.** Verfahren zum Reinigen eines Zielmaterials, umfassend die Verwendung des Affinitätschromatographieträgers nach einem der Ansprüche 1 bis 8.

**11.** Verfahren nach Anspruch 10, wobei das Zielmaterial ein Zielprotein ist.

**Revendications**

**1.** Support de chromatographie d'affinité, le support comprenant :

un support solide comprenant un copolymère comprenant (M-1) de 40 parties en masse ou plus à 80 parties en masse ou moins d'un motif structurel dérivé d'un monomère monovinylique contenant un groupe époxy pour 100 parties en masse de tous les motifs structurels et (M-2) 15 à 60 parties en masse d'un motif structurel dérivé d'un monomère polyvinylique pour 100 parties en masse de tous les motifs structurels, dans lequel le copolymère comprend en outre (M-3) 5 à 25 parties en masse d'un motif structurel dérivé d'un monomère monovinylique sans époxy pour 100 parties en masse de tous les motifs structurels, dans lequel le monomère monovinylique sans époxy est choisi dans l'ensemble constitué par l'éthylvinylbenzène, le mono(méth)acrylate de glycérol et l'hydroxyéthyl(méth)acrylamide ;
des groupes époxy décyclisés obtenus par soumission des groupes époxy à une décyclisation ; et
un ligand couplé au support solide,
le support de chromatographie d'affinité étant tel qu'après qu'un certain récipient formant colonne a été garni du support et ensuite purgé avec un tampon phosphate de sodium 20 mM contenant du NaCl 2 M ayant un pH de 7,5, le fait de laisser la colonne reposer à 40°C pendant 7 jours augmente le pH dans la colonne d'au plus 2, et les groupes époxy décyclisés étant représentés par la formule (1) :

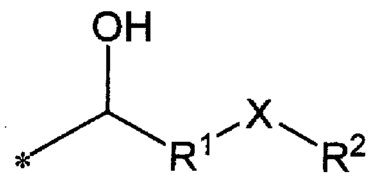

(1)

dans laquelle $R^1$ représente un groupe organique divalent de 1 à 6 atomes de carbone, $R^2$ représente un atome d'hydrogène ou un groupe organique monovalent de 1 à 10 atomes de carbone, X représente un groupe thio (>S), un groupe sulfinyle (>S=O), un groupe sulfonyle (>S(=O)$_2$), un groupe imino (>NH), ou un groupe oxy (>O), et * représente une position de liaison.

2. Support de chromatographie d'affinité selon la revendication 1, dans lequel $R^2$ est représenté par la formule (2) :

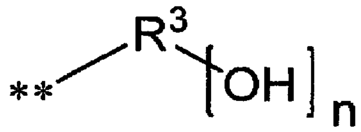

(2)

dans laquelle $R^3$ représente un groupe organique divalent ou trivalent de 1 à 10 atomes de carbone, n vaut 1 ou 2, et ** représente une position de liaison avec X dans la formule (1).

3. Support de chromatographie d'affinité selon la revendication 1 ou 2, dans lequel X est un groupe thio (>S), un groupe sulfinyle (>S=O) ou un groupe oxy (>O).

4. Support de chromatographie d'affinité selon l'une quelconque des revendications 1 à 3, dans lequel X est un groupe thio (>S) ou un groupe sulfinyle (>S=O).

5. Support de chromatographie d'affinité selon l'une quelconque des revendications 1 à 4, dans lequel le ligand est une protéine de liaison à une immunoglobuline.

6. Support de chromatographie d'affinité selon la revendication 5, dans lequel la protéine de liaison à une immunoglobuline est une ou plusieurs choisies dans l'ensemble constitué par la protéine A, la protéine G, la protéine L, la protéine se liant à Fc, et leurs variants fonctionnels.

7. Support de chromatographie d'affinité selon l'une quelconque des revendications 1 à 6, dans lequel le support solide est sous la forme d'une particule poreuse.

8. Support de chromatographie d'affinité selon la revendication 7, dans lequel la particule poreuse a une granulométrie moyenne en volume, déterminée par un analyseur de distribution de granulométrie à diffusion diffractive de laser, de 35 à 100 μm.

9. Colonne de chromatographie d'affinité comprenant : un récipient formant colonne ; et le support de chromatographie d'affinité selon l'une quelconque des revendications 1 à 8 avec lequel le récipient formant colonne est garni.

10. Procédé pour purifier un matériau cible, comprenant l'utilisation du support de chromatographie d'affinité selon l'une quelconque des revendications 1 à 8.

11. Procédé selon la revendication 10, dans lequel le matériau cible est une protéine cible.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008523140 W
- JP 2009522580 W
- JP 8278299 A
- JP 10501173 W
- WO 2011125674 A
- JP 2012141212 A
- US 2014005357 A1
- EP 2339339 A1
- JP 57024369 B